(19) **Europäisches Patentamt**

**European Patent Office**

**Office européen des brevets**

(11) **EP 1 315 693 B1**

(12) **EUROPEAN PATENT SPECIFICATION**

(45) Date of publication and mention
of the grant of the patent:
**02.11.2005 Bulletin 2005/44**

(21) Application number: **01960925.4**

(22) Date of filing: **22.08.2001**

(51) Int Cl.[7]: **C07C 45/79**, C07C 45/50,
B01J 31/40, B01J 41/04,
C01G 55/00, C01G 51/00,
C22B 3/00

(86) International application number:
**PCT/GB2001/003780**

(87) International publication number:
**WO 2002/020451 (14.03.2002 Gazette 2002/11)**

(54) **RECOVERY OF METALS FROM OLEFIN HYDROFORMYLATION REACTIONS**

METALLWIEDERGEWINNUNG AUS OLEFINHYDROFORMYLIERUNGSREAKTIONEN

RECUPERATION DE METAUX A PARTIR DE PROCEDES D'HYDROFORMYLATION

(84) Designated Contracting States:
**AT BE CH CY DE DK ES FI FR GB GR IE IT LI LU MC NL PT SE TR**

(30) Priority: **05.09.2000 GB 0021715**

(43) Date of publication of application:
**04.06.2003 Bulletin 2003/23**

(73) Proprietor: **Johnson Matthey PLC**
**London SW1Y 5BQ (GB)**

(72) Inventors:
  • **BROWN, Alistair, Chalmers, Ramsay**
    **Darlington, County Durham DL3 9AE (GB)**
  • **PEARCE, Ronald**
    **Yarm, Cleveland TS15 9JZ (GB)**
  • **REYNOLDS, Geoffrey**
    **Redcar, Yorkshire TS10 3QD (GB)**
  • **BURNHAM, David, Robert**
    **Macclesfield, Cheshire SK11 8DD (GB)**
  • **PICKARD, Derek, John**
    **Stockton on Tees, Cleveland (GB)**

(74) Representative: **Gibson, Sara Hillary Margaret**
**Synetix Intellectual Property Dept.,**
**Building N, Room N101, Chilton Site,**
**P.O. Box 1,**
**Belasis Avenue**
**Billingham, Cleveland TS23 1LB (GB)**

(56) References cited:
**GB-A- 1 321 275**          **US-A- 5 208 194**

• **DATABASE COMPENDEX [Online]**
**ENGINEERING INFORMATION, INC., NEW YORK, NY, US; SAPJETA JOYCE ET AL: "REGENERATION OF ION-EXCHANGE RESINS USED FOR GOLD RECOVERY" Database accession no. EIX87110173933 XP002181626 & IND ENG CHEM RES AUG 1987, vol. 26, no. 8, August 1987 (1987-08), pages 1716-1719,**

**Description**

This invention relates to the recovery of rhodium or cobalt catalyst values from olefin hydroformylation reactions.

Alcohols, particularly relatively long chain alcohols, are made industrially by the catalysed hydroformylation of olefins with carbon monoxide and hydrogen at elevated temperature and pressure. The reaction is normally carried out in two stages, first the hydroformylation of the olefin to give the $C(n + 1)$ aldehyde followed by hydrogenation of the aldehyde to the alcohol as follows:

$$\text{(i)} \quad RCH{=}CH_2 + CO + H_2 \rightarrow RCH_2CH_2CHO$$

$$\text{(ii)} \quad RCH_2CH_2CHO + H_2 \rightarrow RCH_2CH_2CH_2OH$$

Typically, the catalysts used are transition metals, particularly cobalt or rhodium. The catalyst can be supplied to the reaction as a simple metal salt e.g. as a carboxylate salt dissolved in some of the feed olefin. For rhodium, the active catalyst species is not known although it is probably a carbonyl complex of the metal. Each of the two catalyst metals has particular advantages over the other and the choice in any particular case will be based on a combination of the desired catalyst properties, including the activity of the catalyst , the cost of providing the metal to the system and the selectivity to the required products. Rhodium has the advantage of being a more active catalyst than cobalt and it is more selective, giving a cleaner product. In particular, rhodium is a much less active hydrogenation catalyst for the intermediate aldehyde so the aldehyde can be recovered from the initial reaction in a relatively pure form. The alcohol can be formed by hydrogenation of the aldehyde in a second reaction. When cobalt is used as the catalyst, part of the aldehyde is typically hydrogenated to the alcohol. However, this hydrogenation is not complete so a separate hydrogenation step is still required. A disadvantage of using a rhodium catalyst is that it is much more expensive than cobalt and this makes it important to the economics of the process to recover the metal from the product. This remains true even though the high activity of the rhodium catalyst means that the level of catalyst in the product is typically only a few ppm w/w (parts per million by weight). The use of rhodium has been inhibited by the absence of practical means of recovering substantially all the rhodium values from such a dilute product stream.

GB-A-1321275 describes a recovery process from such a reaction product stream in which rhodium is separated by absorption of a particular metal carbonyl hydride on a basic ion exchange resin. The separation method requires that the pressure be kept well above ambient pressure in order to ensure that the rhodium carbonyl hydride complex is present.

The need to operate at such high pressures, necessitating complex and expensive vessels, valves and piping for industrial scale operation, may have limited the application of this process. No method for recovering the rhodium from the resin is described.

The value of rhodium means that economic operation of the process necessitates recovery of the rhodium from the spent resin. There is therefore a requirement for an economical method of recovering a metal such as rhodium from an ion exchange resin onto which a compound of the metal has been absorbed. This is not as straightforward as in most ion exchange resin regeneration processes. We have found that it is very difficult to remove the rhodium values from the spent resin by simple ion exchange. Without wishing to be bound by the theory, we believe it likely that the rhodium species, as initially extracted onto the resin, is insoluble or that it is converted into the insoluble species subsequent to the extraction. Whatever the reason, the important practical consequence is that techniques other than those normally used for recovery from an ion exchange resin are necessary.

GB-A-1355209 describes a method for recovering rhodium from an ion exchange resin by treating the rhodium-loaded resin with a lower alkanol, water, a water-soluble aliphatic amine and oxygen.

GB-A-1576514 describes a process in which a distillation residue from the product stream of a rhodium-catalysed hydroformylation process is treated with an oxygen-containing mineral acid and a peroxide and the resulting rhodium salt is then treated with an ion exchanger. The ion exchange resin is then further treated with hydrochloric acid to desorb the rhodium ions which are then reacted with e.g. carbon monoxide in the presence of an alkyl phosphine and a water-soluble organic solvent to reform the rhodium catalyst for the hydroformylation reaction.

DE 2045415 describes a process for recovering cobalt which is in the form of a cobalt carbonyl on a basic ion exchange resin by treating the cobalt carbonylate loaded ion exchange resin with a mixture of a lower alkanol and an aqueous alkali.

US-A-4388279 describes removing trace amounts of catalysts which are present in products resulting from organic reactions, such as rhodium which is present in the alcohol products resulting from a hydroformylation reaction, by treating the aforesaid products with a solid adsorbent such as a metal compound of Groups IA or IIA of the Periodic Table, molecular sieves or ion-exchange resins at a temperature in the range of from about ambient to about 100°C and a pressure in the range of from about atmospheric to about 100 atmospheres.

The reaction mixture containing alcohol products must first be contacted with a stripping agent such as aqueous ammonium hydroxide, anhydrous ammonia or an amine compound. Upon completion of the extraction or treatment period, the stripping agent solution containing substantially all of the rhodium complex catalyst is separated from the organic phase which comprises the alcohol product and only the stripped alcohol stream containing trace residual rhodium is contacted with the solid adsorbent. No method of recovering the rhodium metal from an ion exchange resin is suggested.

US-A-5208194 describes a process for recovering a liganded rhodium hydrocarbyl complex from an organic solution by contact with an acidic ion exchange resin that has sulfonic acid active groups.

It is an object of the present invention to provide an alternative method for recovering rhodium and/ or cobalt metal values, especially for recovering rhodium values, from hydroformylation reactions.

It is a further object of the present invention to provide an alternative method for recovering rhodium and/or cobalt metal values from an ion exchange resin to which said metal values are bound.

Accordingly, the present invention provides a method of recovering rhodium and/or cobalt metal values from a hydroformylation reaction product stream comprising the steps of:

a) contacting the reaction product stream with a basic ion exchange resin such that at least a part of the metal is bound to the resin as metal species,
b) incinerating said basic ion exchange resin containing the metal species to produce an ash containing the metal and / or the metal oxides and
c) separating said metal and/or oxides from the remainder of the ash.

In a second aspect of the invention, we provide a method of recovering rhodium and/or cobalt metal values from an ion exchange resin to which a metal species is bound comprising incinerating said resin to produce an ash containing the metal and / or the metal oxides and separating said metal and/or oxides from the remainder of the ash.

The method of the invention is particularly useful for recovering rhodium or cobalt values from a hydroformylation reaction product stream Therefore the metals are rhodium and cobalt.

In a still further aspect of the present invention we provide a hydroformylation process comprising:

a) reacting an olefin with carbon monoxide and hydrogen at super-ambient temperature and pressure in the presence of a homogeneous catalyst comprising rhodium and/or cobalt to form a reaction product stream containing said rhodium and/or cobalt catalyst, and
b) contacting at least a part of the reaction product stream with a basic ion exchange resin at a pressure not exceeding 5 bar gauge and a temperature of not more than 120°C such that at least a part of the rhodium and/or cobalt therein is bound to the resin.

The homogeneous rhodium catalyst is preferably an unliganded rhodium compound. Preferably the reaction product stream is not treated with an ammonia or amine compound as an intermediate step between the hydroformylation step and contact with the resin. We have found such a step which is described in prior art processes for removing most of the metal values from the product stream, to be unnecessary.

The process preferably comprises the additional steps of incinerating said basic ion exchange resin containing the bound rhodium species to produce an ash containing the rhodium as metal and / or the metal oxides; separating the rhodium and/or oxides from the remainder of the ash, and then, optionally, converting the separated rhodium or oxides into a form which may be used as a homogeneous catalyst for the hydroformylation reaction.

The reaction product stream is optionally passed through a hydrogenation reaction step and/or separated into separate product streams, usually subsequent to the contact with the ion exchange resin. This is because any further processing of the liquid product stream which necessitates vapourisation, for example to effect a vapour-phase hydrogenation or separation process, would tend to leave some of the metal deposited on the process equipment and therefore would tend to reduce the amount of metal which could be readily recovered from the product stream. The invention, can of course be applied to treat a product stream which has undergone further process steps between steps (a) and (b) above and therefore such processes are not excluded from the scope of the invention.

The ion exchange resin to which a rhodium or cobalt species is bound is derived from a hydroformylation process in which a rhodium or cobalt containing stream is treated with an ion exchange resin. The prior art processes require that a product stream from a hydroformylation reaction is pre-treated before contacting it with the ion exchange resin or that the treatment of such a stream with the ion exchange resin is carried out at greatly elevated pressures so that the metal species is present as a metal carbonyl hydride. We have found that, contrary to the teaching of GB 1321275, rhodium can be extracted from hydroformylation reaction product streams under conditions such that the complex required by GB 1321275 would not be present. Suitable conditions are near ambient pressure and near ambient or moderately superambient temperature and extraction of the rhodium is very nearly quantitative. We have successfully reduced the concentration of rhodium in the reaction product stream from about 5 ppm to about 0.1 ppm using fresh ion exchange resin. This represents 98% recovery, but is likely to diminish as the remaining capacity of the ion exchange resin is diminished.

Accordingly, in a further aspect of the present invention we provide a method of removing rhodium values from a reaction product stream which comprises contacting the reaction product stream with a basic ion exchange resin at a pressure not exceeding 5 bar gauge and a temperature of not more than 120°C such that at least a part of the rhodium is bound to the resin and subsequently incinerating said basic ion exchange resin containing the rhodium species to produce an ash containing rhodium metal and / or rhodium oxides and separating said rhodium and/or oxides from the remainder of the ash.

The ion exchange resin is a strongly or weakly basic resin. We have found that acidic resins are not suitable because the recovery using an acidic resin is poor. Typically these resins contain amino-groups as the active absorption site, normally tertiary amine or quaternary ammonium salt. In the latter case it may be preferred to avoid using a resin containing e.g. chloride ions which may interact with the plant materials. In this case the anion may be exchanged to e.g. a hydroxy ion. Examples of particular useful resins include Amberlyst™ A21, A26 and A27 from Rohm and Haas.

The temperature and pressure conditions are stated above as being near ambient. The pressure is less than 5 bar gauge, but will usually be less than 4 bar gauge, more typically less than 2 bar gauge. The pressure may be reduced, e.g. to < 1 bar g, before it is increased to the operating pressure to facilitate degassing of the liquid product stream prior to contact with the resin. An elevated pressure helps to prevent any dissolved gases from coming out of solution and causing channelling in the resin bed and also facilitates the flow of reaction product stream through the bed. Typically the pressure drop across a column of resin used in the invention will be from 0.2 to 0.5 bar. The pressure drop across a fresh column is usually less than across one that is nearly spent, probably because of settling in the column and/ or trapping of fines within the column.

The ion exchange resin absorbs rhodium more rapidly at higher temperatures and thus the absorption process will usually be carried out at temperatures above ambient. However, the rhodium species in the aldehyde product stream of a hydroformylation reaction are not stable at ambient pressures at temperatures above about 120°C. In practice, a balance is struck between speed of absorption and stability and temperatures of from 20 to 100°C, particularly 70 to 80°C, are most suitable.

The form in which the rhodium is extracted by the ion exchange resin is not the hydrido carbonyl species as described in GB 1321275 as this complex requires much higher partial pressures of hydrogen and/ or carbon monoxide to be present in significant concentrations (relative to the total amount of rhodium present) as described by J.L Vidal & W.E. Walker, Inorg Chem, 1981 20 pp 249 - 254. The increase in absorption speed with temperature noted above indicates a definite activation energy for the species formed on the resin. Without being bound by theory, we infer from this that the absorption process is not a simple ion exchange process.

It appears that the rhodium is present in the product stream as a species that is changed on exposure to the atmosphere, presumably by oxidation and the resulting oxidised forms of rhodium are not efficiently extracted by the ion exchange resin. Therefore it is highly preferable to avoid exposing the reaction product stream to the atmosphere, or other sources of oxidation, before the rhodium is extracted from it. In practice, the reaction product stream from a hydroformylation reaction is not deliberately exposed to the atmosphere until after any subsequent hydrogenation reaction to generate alcohol product.

In typical industrial operation a number of ion exchange columns will be connected together so that the product stream can be directed to one or more of several columns so that spent columns can be removed from use and filled with fresh resin ready for re-use. Although the pipework and valves required to do this will inevitably be fairly complex, it is a major advantage of the present invention over GB 1321275 that the operation is not carried out at a high pressure.

We have found that a particularly convenient arrangement is to have three columns and associated pipework and valves so that any two columns can be connected in flow series and the remaining column isolated. In this type of operation the first and second columns are connected and used in series whilst the third is isolated and the spent resin removed and replaced with fresh resin. The size and capacity of each column will typically be chosen such that one column is capable of extracting rhodium from the reaction product stream to give an output concentration close to zero e.g. less than 0.5 ppm and usually about 0.1 ppm, for a period of several days and preferably more than two weeks. In practice therefore the size and capacity of each column and the number of columns selected depends upon the amount of rhodium-containing product stream which is to be treated. A different number and arrangement of columns may be preferred, depending upon the economic evaluation of the process from which rhodium recovery is desired.

In an example operation of the method of this aspect of the invention, first and second columns are operated until the upstream column is spent such that a significant concentration of rhodium values is passing to the second column. At this point, the third column is connected downstream of the second column and the first isolated for removal and replacement of the resin and the process repeated to enable substantially continuous rhodium extraction. Using two columns in series means that the upstream column can be used to near complete capacity whilst the downstream column is still relatively fresh. As the first column has absorbed rhodium to near its full capacity, the second column remains relatively fresh and is capable of absorbing substantially all the rhodium in the product stream flowing from the first column. The time needed to remove and replace spent resin from the isolated column is typically much shorter than the practical life of a column in use so arranging substantially continuous operation is not difficult.

The resin burns easily and it is preferred to control the incineration in such a way as to prevent the uncontrolled release of particulates from the process. Therefore the resin is preferably dried, usually in flowing air and at a temperature in the range 100 - 500 °C. The drying process should be done with care in order to avoid spitting which would result in loss of the resin. The resin may then be ignited at from about 500 to 600 °C and then the incineration of the metal-containing resin is carried out at temperatures from about 600 to about 850 °C, for example up to about 800°C. The resin is normally loaded in shallow trays to give maximum exposure to the air and promote combustion. When the incineration is complete the equipment is allowed to cool and the ash is collected in preparation for separation of the desired metals and subsequent reconversion to the catalytic form, if desired.

As typical basic ion exchange resins are based on aromatic ring containing polymers such as polystyrene, a suitable commercial scale incinerator preferably includes a secondary combustion stage to ensure combustion of the smoke from the initial incineration. A suitable incinerator therefore includes a primary combustion chamber in which the resin is burned. Air is blown through this chamber and an oil or gas fired heater is used to initiate the combustion and raise the temperature to the combustion temperature. The off-gas from the primary combustion chamber may then pass through a secondary combustion chamber maintained at about 800°C by an oil or gas fired heater. An example of suitable equipment is an Evans Universal 'Maximaster' II incinerator.

The residual ash contains the recovered metal values, e.g. rhodium or cobalt, as well as other inorganic material such as metallic species from the reactor plant itself or other residues from the exchange resin. The recovered metal species is normally present in the ash as the metal or as an oxide, or most likely a mixture containing both. Methods of separating the recovered metal compounds from the ash are well known to those skilled in the art of metals processing and will depend upon the nature of the metal and the form in which it is required to be recovered.

The metal, e.g. rhodium or cobalt present in the ash may be purified and converted back to the catalyst from which it was derived initially or alternatively into another form of the metal. In a preferred method, rhodium or cobalt oxides are converted to a rhodium or cobalt compound which is an active catalyst for the hydroformylation of olefins. For example, when the metal is rhodium and it is required for re-use in a hydroformylation reaction, it can be separated from the ash by treating the ash with aqueous HCl and chlorine, optionally after a reduction step to convert Rh oxides to Rh metal, to give $RhCl_3$ which, after purification if necessary, can be converted into an olefin-soluble carboxylate salt e.g. rhodium stearate, by reaction with a carboxylate metal e.g. sodium, salt. The precipitated rhodium carboxylate can then be dissolved in olefin feedstock for reuse as catalyst. Using this recovery technique, we have successfully returned more than 90% of the rhodium fed to the hydroformylation for reuse as catalyst.

In practice it may be convenient for the recovery of the metal from the ash to be carried out by a specialist metals processing operator using techniques specific to the precious metals industry.

As an example, the rhodium oxide recovered from the incineration of the ion exchange resin may be converted by known methods to a rhodium "sponge" i.e. a highly porous form of rhodium before further processing to the desired rhodium compound or metal.

The following Examples illustrate the invention. All parts and percentages are by weight unless otherwise stated.

Example 1

A rhodium extraction column was set up as follows. Basic ion exchange resin, Amberlyst A 21, was washed with dry methanol to remove water and dried in a current of nitrogen. 22.5 ml of the dry resin (about 6 g) was placed in a vertical dry glass column, fitted with an outer water jacket supplied from a thermostatically controlled water bath and having an internal diameter of 6 mm, and maintained under a dry nitrogen atmosphere at a pressure of 2 inches water gauge (about 5 mbar).

An aldehyde reaction product, prepared by the hydroformylation of a mixed heptenes feedstock in the presence of rhodium as a homogeneous catalyst and containing about 80% C8 aldehydes, the remainder being predominantly unreacted olefin and paraffinic by-products, was used as feed to the extraction column. The aldehyde reaction product was preheated to 80°C at the inlet to the extraction column. The temperature of the column was maintained at 80°C by passing water at 80°C through the water jacket. To start up the column the reaction product stream was fed to the column upflow to expand the bed by about 50% by volume and remove gas bubbles. Extraction was then carried out downflow, keeping the liquid level in the column above the top of the resin to maintain the resin in a flooded condition. The flow rate of the aldehyde product stream was about 450 ml per hour. Extraction was continued for a period of 27 days and the concentration of rhodium in the input and outlet streams monitored at intervals. The results are set out in Table 1 below.

Table 1

| Time on line (days) | Rhodium Concn (ppm w/w) | |
|---|---|---|
| | Feed stream | Exit stream |
| 1 | 5.3 | 0.1 |
| 5 | 5.3 | 0.2 |
| 10 | 5 | 0.4 |
| 15 | 5.3 | 0.4 |
| 20 | 4.8 | 0.9 |
| 25 | 4.8 | 1.2 |
| 27 | 4.8 | 1.2 |

The data in Table 1 show that efficient extraction of rhodium was maintained for a period of at least 15 days. The rhodium content of the resin removed from the column after the 27 day operation period was 1012 mg, equivalent to 4.5 g rhodium per 100 ml of dry resin (about 160 g per kg of resin). The relatively long life of the resin enables multi-column, particularly three column, operation as generally described above, to be implemented relatively simply.

Example 2

Rhodium values were extracted from an aldehyde reaction stream from the hydroformylation of $C_{12}$ to $C_{14}$ olefins carried out using rhodium as homogeneous catalyst. The extraction column used was generally as described in Example 1, but of larger size, internal diameter 13 mm, and using a resin charge of 87 ml (about 24 g). The feed to the extraction column contained about 95% $C_{13-15}$ aldehyde from a continuous synthesis vessel. The feed was reduced to ambient pressure in a degassing vessel and pumped to the extraction column with the exclusion of air. The column temperature was controlled at 70°C and the flow rate was 808 ml.hr$^{-1}$. The extraction was continued for 11 days over which time the rhodium concentration in the feed stream to the column varied between 2.9 and 4.7 ppm and the concentration in the exit stream was constant at 0.1 ppm throughout.

Example 3

An aldehyde reaction product, prepared by the hydroformylation of a mixed heptenes feedstock in the presence of rhodium and cobalt as a mixed homogeneous catalyst and containing about 80% $C_8$ aldehydes, the remainder being predominantly unreacted olefine and paraffinic by-products, was used as feed to an extraction column constructed as described in Example 1. The extraction temperature was 80°C. The concentration of the rhodium was reduced from 5 ppm to 0.15 ppm. The concentration of the cobalt was reduced from 15 ppm to 4 ppm.

Example 4

The rhodium present in spent basic ion exchange resin, produced under conditions similar to those described in Examples 1 and 2, was recovered by incinerating the resin in a two-chamber oil fired incinerator (Evans Universal "Maximaster II"). The resin was washed with methanol to remove the residual aldehyde and then with water to remove the methanol. The wet resin (10 kg) was spread out in a steel tray with the dimensions 300 mm x 850 mm x 75 mm and placed in the primary combustion chamber of the incinerator. Initially the resin was dried by heating it to 500°C in a stream of air (90 m$^3$/hour) for 35 minutes. During the drying period, close observation showed that there was no loss of resin from the tray by 'spitting' or thermal shock. The temperature was then raised to 580°C over 15 minutes and the resin ignited. The temperature in the primary chamber reached 800°C after a further 15 minutes and was maintained at 700 - 800°C for a further 45 minutes to complete the incineration. During the incineration the temperature in the secondary combustion chamber was maintained at about 800 °C with an air flow of 70 m$^3$/hour in order to burn the decomposition products from the primary combustion. When the incineration was complete the ash was allowed to cool. The ash contained 65% Rh as metal plus metal oxide as well as traces of other metals (such as iron) and their oxides.

The ash was then treated with HCl and chlorine to give aqueous rhodium trichloride. Catalytically useful rhodium was obtained by reaction with sodium stearate solution. The rhodium stearate precipitated from the solution, and was separated and dissolved in olefin feed to form a stock solution for use as a catalyst in further hydroformylation reactions.

**Claims**

1. A hydroformylation process comprising the steps of:

   a) reacting an olefin with carbon monoxide and hydrogen at super-ambient temperature and pressure in the presence of a homogeneous catalyst comprising rhodium or cobalt or a mixture of rhodium and cobalt to form a reaction product stream containing said rhodium and/or cobalt catalyst, and
   b) contacting at least a part of the reaction product stream with a basic ion exchange resin at a pressure not exceeding 5 bar gauge and a temperature of not more than 120°C such that at least a part of the rhodium and/or cobalt therein is bound to the resin.

**2.** A hydroformylation process as claimed in claim 1 further comprising the steps of

c) incinerating said basic ion exchange resin containing the bound rhodium and/or cobalt species to produce an ash containing the rhodium and/or cobalt as metal and / or the metal oxides,

d) separating said metal and/or oxides from the remainder of the ash, and

e) optionally converting said separated metal and/ or oxides into a form which may be used as a homogeneous catalyst for step (a).

**3.** A hydroformylation process as claimed in claim 1 or claim 2, further comprising passing the reaction product stream through a hydrogenation reaction step and/or a separation step subsequent to the contact with the ion exchange resin.

**4.** A hydroformylation process as claimed in any of the preceding claims, wherein the reaction product stream is contacted with the basic ion exchange resin at a pressure less than 5 bar gauge.

**5.** A hydroformylation process as claimed in any of the preceding claims, wherein the reaction product stream is contacted with the basic ion exchange resin at a pressure less than 4 bar gauge.

**6.** A hydroformylation process as claimed in any of the preceding claims, wherein the reaction product stream is contacted with the basic ion exchange resin at a pressure not exceeding 2 bar gauge.

**7.** A hydroformylation process as claimed in any of the preceding claims, wherein the reaction product stream is degassed before contact with the ion exchange resin.

**8.** A hydroformylation process as claimed in any of the preceding claims, wherein at least two ion exchange columns are connected together in such a way that the reaction product stream can be directed to one or more of said columns and at least one column may be isolated from the flow of the product stream.

**9.** A hydroformylation process as claimed in any of the preceding claims, wherein at least two ion exchange columns are connected together so that they operate in series.

**10.** A hydroformylation process as claimed in any of claims 2 - 9, wherein the resin is dried at a temperature between 100 and 500 °C prior to incineration.

**Patentansprüche**

**1.** Hydroformylierungsverfahren, das die Schritte umfasst:

a) Umsetzen eines Olefins mit Kohlenmonoxid und Wasserstoff bei einer Temperatur und einem Druck über Umgebungsbedingungen und in Gegenwart eines homogenen Katalysators, der Rhodium oder Kobalt oder eine Mischung aus Rhodium und Kobalt umfasst, um einen Strom eines Reaktionsprodukts zu bilden, der den Rhodium- und/oder Kobalt-Katalysator enthält, und

b) Inkontaktbringen wenigstens eines Teils des Stroms des Reaktionsprodukts mit einem basischen Ionenaustauscherharz bei einem Druck, der 5 bar Überdruck nicht überschreitet, und bei einer Temperatur von nicht mehr als 120°C, so dass wenigstens ein Teil des Rhodiums und/ oder Kobalts darin an das Harz gebunden wird.

**2.** Hydroformylierungsverfahren nach Anspruch 1, das außerdem die Schritte umfaßt:

c) Veraschen des basischen Ionenaustauscherharzes, das die gebundene Rhodium- und/oder Kobalt-Spezies enthält, um eine Asche zu erzeugen, die das Rhodium und/oder Kobalt als Metall und/oder Metalloxide enthält,

d) Abtrennen des Metalls und/oder der Oxide vom Rest der Asche, und

e) gegebenenfalls Umwandeln des abgetrennten Metalls und/oder der Oxide in eine Form, die als homogener Katalysator für Stufe (a) verwendet werden kann.

**3.** Hydroformylierungsverfahren nach Anspruch 1 oder Anspruch 2, das im Anschluss an den Kontakt mit dem Ionenaustauscherharz außerdem das Hindurchleiten des Stroms des Reaktionsprodukts durch eine Stufe einer Hydrierreaktion und/oder eine Trennstufe umfasst.

**4.** Hydroformylierungsverfahren nach irgendeinem der vorausgehenden Ansprüche, wobei der Strom des Reaktionsprodukts mit dem basischen Ionenaustauscherharz bei einem Druck von weniger als 5 bar Überdruck in Kontakt gebracht wird.

**5.** Hydroformylierungsverfahren nach irgendeinem der vorausgehenden Ansprüche, wobei der Strom des Reaktionsprodukts mit dem basischen Ionenaustauscherharz bei einem Druck von weniger als 4 bar Überdruck in Kontakt gebracht wird.

**6.** Hydroformylierungsverfahren nach irgendeinem der vorausgehenden Ansprüche, wobei der Strom des Reaktionsprodukts mit dem basischen Ionenaustauscherharz bei einem Druck in Kontakt gebracht wird, der 2 bar Überdruck nicht überschreitet.

7. Hydroformylierungsverfahren nach irgendeinem der vorausgehenden Ansprüche, wobei der Strom des Reaktionsprodukts vor dem Kontakt mit dem Ionenaustauscherharz entgast wird.

8. Hydroformylierungsverfahren nach irgendeinem der vorausgehenden Ansprüche, wobei wenigstens zwei Ionenaustauscherkolonnen so miteinander verbunden sind, dass der Strom des Reaktionsprodukts einer oder mehreren der genannten Kolonnen zugeführt werden kann und wenigstens eine Kolonne gegenüber dem Durchfluß des Produktstroms isoliert werden kann.

9. Hydroformylierungsverfahren nach irgendeinem der vorausgehenden Ansprüche, wobei wenigstens zwei Ionenaustauscherkolonnen so miteinander verbunden sind, dass sie in Reihe arbeiten.

10. Hydroformylierungsverfahren nach irgendeinem der Ansprüche 2 bis 9, wobei das Harz vor der Veraschung bei einer Temperatur zwischen 100 und 500°C getrocknet wird.


**Revendications**

1. Procédé d'hydroformylation, comprenant les étapes consistant :

a) à faire réagir une oléfine avec du monoxyde de carbone et de l'hydrogène à une température et une pression supérieures aux valeurs ambiantes en présence d'un catalyseur homogène comprenant du rhodium ou du cobalt ou un mélange de rhodium et de cobalt pour former un courant de produit de réaction contenant ledit catalyseur au rhodium et/ou cobalt, et
b) à mettre en contact au moins une partie du courant de produit de réaction avec un résine échangeuse d'ions basique à une pression non supérieure à 5 bars au manomètre et une température non supérieure à 120°C de telle sorte qu'au moins une partie du rhodium et/ou cobalt qui s'y trouve soit liée à la résine.

2. Procédé d'hydroformylation suivant la revendication 1, comprenant en outre les étapes consistant :

c) à incinérer ladite résine échangeuse d'ions basique contenant l'entité au rhodium et/ou cobalt liée pour produire une cendre contenant le rhodium et/ou cobalt sous forme de métal et/ou des oxydes métalliques,
d) à séparer ledit métal et/ou lesdits oxydes du reste de la cendre, et
e) facultativement à convertir ledit métal séparé et/ou lesdits oxydes séparés sous une forme qui peut être utilisée comme catalyseur homogène pour l'étape (a).

3. Procédé d'hydroformylation suivant la revendication 1 ou la revendication 2, comprenant en outre le passage du courant de produit de réaction par une étape de réaction d'hydrogénation et/ou une étape de séparation après le contact avec la résine échangeuse d'ions.

4. Procédé d'hydroformylation suivant l'une quelconque des revendications précédentes, dans lequel le courant de produit de réaction est mis en contact avec la résine échangeuse d'ions basique à une pression inférieure à 5 bars au manomètre.

5. Procédé d'hydroformylation suivant l'une quelconque des revendications précédentes, dans lequel le courant de produit de réaction est mis en contact avec la résine échangeuse d'ions basique à une pression inférieure à 4 bars au manomètre.

6. Procédé d'hydroformylation suivant l'une quelconque des revendications précédentes, dans lequel le courant de produit de réaction est mis en contact avec la résine échangeuse d'ions basique à une pression ne dépassant pas 2 bars au manomètre.

7. Procédé d'hydroformylation suivant l'une quelconque des revendications précédentes, dans lequel le courant de produit de réaction est dégazé avant mise en contact avec la résine échangeuse d'ions.

8. Procédé d'hydroformylation suivant l'une quelconque des revendications précédentes, dans lequel au moins deux colonnes échangeuses d'ions sont connectées ensemble de telle sorte que le courant de produit de réaction puisse être dirigé à une ou plusieurs desdites colonnes et au moins une colonne puisse être isolée du flux du courant de produit.

9. Procédé d'hydroformylation suivant l'une quelconque des revendications précédentes, dans lequel au moins deux colonnes échangeuses d'ions sont connectées ensemble de telle sorte qu'elles fonctionnent en série.

10. Procédé d'hydroformylation suivant l'une quelconque des revendications 2 à 9, dans lequel la résine est séchée à une température comprise dans l'intervalle de 100 à 500°C avant incinération.